(19) Europäisches Patentamt
European Patent Office
Office européen des brevets

(11) EP 0 622 352 B1

# (12) EUROPÄISCHE PATENTSCHRIFT

(45) Veröffentlichungstag und Bekanntmachung des Hinweises auf die Patenterteilung:
**27.08.1997 Patentblatt 1997/35**

(51) Int Cl.$^6$: **C07C 67/04**, C07C 69/01

(21) Anmeldenummer: **94106106.1**

(22) Anmeldetag: **20.04.1994**

## (54) Verfahren zur Herstellung von Carbonsäurevinylestern

Process for the preparation of vinyl esters of carboxylic acids

Procédé de fabrication d'esters vinyliques d'acides carboxyliques

(84) Benannte Vertragsstaaten:
**BE CH DE FR GB LI NL**

(30) Priorität: **28.04.1993 DE 4313922**

(43) Veröffentlichungstag der Anmeldung:
**02.11.1994 Patentblatt 1994/44**

(73) Patentinhaber: **BASF Aktiengesellschaft
67063 Ludwigshafen (DE)**

(72) Erfinder:
- **Heider, Dr.Marc
67433 Neustadt (DE)**
- **Ruehl, Dr. Thomas
67227 Frankenthal (DE)**
- **Henkelmann, Dr. Jochem
68165 Mannheim (DE)**

(56) Entgegenhaltungen:
**EP-A- 0 512 656**

- **JOURNAL OF ORGANIC CHEMISTRY, Bd. 52, Nr. 11, 29. Mai 1987, Easton, US, Seiten 2230-2239, T. MITSUDO et al, "Ruthenium-catalyzed selective addition of carboxylic acids to alkynes. A novel synthesis of enol esters"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 622 352 B1

## Beschreibung

Die vorliegende Erfindung betrifft ein verbessertes Verfahren zur Herstellung von Carbonsäurevinylestern durch Umsetzung einer Carbonsäure mit Acetylen in Gegenwart eines Platinmetallkomplex-Katalysators.

In der EP-A 512 656 ist ein Verfahren zur Vinylierung von Brönsted-Säuren, darunter von Carbonsäuren, mit Acetylen beschrieben, bei dem Ruthenium-Metall auf einem inerten Träger als heterogener Katalysator fungiert. Das Arbeiten mit heterogenen Katalysatoren bereitet jedoch verfahrenstechnische Schwierigkeisofern der Katalysator nicht in einem Festbett angeordnet ist, welches von dem Gemisch der Einsatzstoffe durchströmt wird. Die Festbettkatalyse ist allerdings nur bei großtechnischen Verfahren wirtschaftlich und in der Regel nur für die Herstellung eines bestimmten Produktes ausgelegt. Möchte man verschiedene Produkte in einer Anlage im Wechsel herstellen, ist hierfür die homogene Katalyse eher geeignet.

Ferner ist aus der Arbeit vom Mitsudo et al. (J. Org. Chem. 52, Seite 2230, [1987]) die Umsetzung von Carbonsäuren mit Alkinen zu den entsprechenden Alkenylestern mit Hilfe von Cyclooctadienyl-Rutheniumkomplexen als Katalysatoren bekannt, in denen Maleinsäureanhydrid und tertiäre Phosphine als weitere Liganden fungieren. Abgesehen davon, daß diese Komplexe nur schwierig zugänglich sind und für Reaktionen im technischen Maßstab kaum in Betracht kommen, erfordern sie im Falle des Acetylens unverhältnismäßig lange Reaktionszeiten. Dieser Arbeit zufolge findet bei der Umsetzung von Methacrylsäure mit Pent-1-in in Gegenwart eines Rutheniumkomplexes und Tributylphosphin als alleinigem Liganden überhaupt keine Reaktion statt.

In Organometallics 2, Seite 1689, [1983], wurde von Rotem et al. über die Umsetzung von Carbonsäuren mit Alkinen in Gegenwart von Rutheniumcarbonyl- oder Rutheniumcarbonylacetat-Komplexen berichtet. Es wurden aber nur substituierte Alkine, nicht jedoch Acetylen selbst zur Umsetzung gebracht.

Der vorliegenden Erfindung lag daher ein verbessertes und universell anwendbares Verfahren zur Vinylierung von Carbonsäuren als Aufgabe zugrunde.

Demgemäß wurde ein Verfahren zur Herstellung von Carbonsäurevinylestern durch Umsetzung einer Carbonsäure mit Acetylen in Gegenwart eines Platinmetallkomplex-Katalysators gefunden, das dadurch gekennzeichnet ist, daß man als Platinmetallkomplex-Katalysator einen Komplex aus einem Platinmetall und den Liganden Kohlenmonoxid, ein Nitril (I), eine tertiäre Stickstoffbase (II), eine tertiäre Phosphorverbindung mit C- und/oder O-organischen Resten (III) oder Gemische dieser Verbindungen enthält.

Die Katalysatoren des erfindungsgemäßen Verfahrens sind Komplexverbindungen der Platinmetalle Ruthenium, Rhodium, Palladium, Osmium, Iridium oder Platin, von denen besonders Ruthenium bevorzugt ist.

Als Katalysatoren eignen sich Komplexe mit einem oder mehreren Metallkernen, die entweder Metall-Metall-Bindungen aufweisen, oder in denen die Metallatome durch Liganden verbunden sind.

In den Komplexen können die Metalle nullwertig sein oder in einer positiven oder negativen Oxidationsstufe vorliegen, und die Koordinationsstellen der Platinmetalle können ganz oder teilweise von den Liganden besetzt sein.

Solche Liganden sind Kohlenmonoxid, die Nitrile I, tertiäre Amine II sowie tertiäre Phosphorverbindungen mit C- und/oder O-organischen Resten III. Charakteristisch für diese Liganden ist, daß sie wegen ihres Elektronenpaares am C-Atom des Kohlenmonoxids, bzw. am N-Atom bzw. P-Atom der definitionsgemäßen Liganden I bis III zur Komplexbildung mit den Platinmetallen befähigt sind.

Die erfindungsgemäß einzusetzenden ein- oder mehrwertigen Nitrile können durch die allgemeine Formel I wiedergegeben werden,

$$R\text{-}C\equiv N \qquad (I)$$

in der R für einen C-organischen Rest steht, und zwar bevorzugt für

- eine $C_1$- bis $C_{18}$-Alkylgruppe, vor allem eine $C_1$- bis $C_4$-Alkylgruppe wie die Methyl-, Ethyl-, n-Propyl- und die n-Butylgruppe,
- eine Cycloalkylgruppe mit 5 bis 7 Ringgliedern, vor allem die Cyclopentyl- und die Cyclohexylgruppe,
- eine Arylgruppe, wie die Naphthyl- und die Phenylgruppe, vor allem die Phenylgruppe,
- eine Aralkylgruppe, besonders bevorzugt mit unverzweigter Alkylkette, wie die Benzylgruppe oder die 2-Phenylethylgruppe.

Beispiele für geeignete Nitrile sind Acetonitril, Propionitril, Benzonitril und Benzylnitril.

Unter den tertiären Stickstoffbasen kommen insbesondere tertiäre Amine der Formel IIa

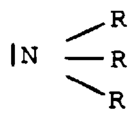

(IIa)

in Betracht, in der die Reste R gleiche oder verschiedene C-organische Gruppen bedeuten, die auch miteinander verbunden sein können. Für die bevorzugten Reste R gilt das gleiche wie für die Reste der Nitrile (I).

Allgemein sind solche tertiären Stickstoffbasen II bevorzugt, deren Reste R insgesamt nicht mehr als 20 C-Atome aufweisen. Bevorzugt sind ferner solche Verbindungen II, die drei gleiche Reste aufweisen oder die zwei gleiche $C_1$- bis $C_4$-Reste und einen $C_5$- bis $C_{18}$-Rest tragen.

Beispiele für gut geeignete Amine IIa sind Trimethylamin, Triethylamin, N,N-Dimethylethylamin, Tri-n-propylamin, Tri-n-butylamin, N,N-Dimethyl-n-octylamin, N,N-Dimethylcyclohexylamin, 1-Methylpyrrolidin, N,N-Dimethylbenzylamin, N,N-Diethylanilin, Triphenylamin und Dimethylstearylamin.

Weiterhin eignen sich als Liganden heteroaromatische Stickstoffbasen (IIb) wie Pyridin, 2,2'-Bipyridyl, Pyrimidin, die unsubstituierten Triazine, Chinolin, Isochinolin, 1,10-Phenanthrolin, Poly-2-vinylpyrrolidon und Purin, bevorzugt Pyridin, 2,2'-Bipyridyl, 1,10-Phenanthrolin und Poly-2-vinylpyridin.

Die tertiären Phosphorverbindungen mit C- und/oder O-organischen Resten können durch die allgemeine Formel III wiedergegeben werden.

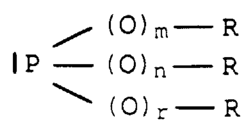

(III)

in der die Gruppen R gleich oder verschieden und C- oder O-organischer Natur sind.

Die Variablen n, m und r können die Werte 0 oder 1 annehmen. Dementsprechend handelt es sich um Phosphine (m, n, r = 0), Phosphinite (eine Variable = 1), Phosphonite (zwei Variablen = 1) oder Phosphite (m, n, r = 1).

Die geeigneten und bevorzugten Reste R haben die gleiche Bedeutung wie im Falle der Nitrile (I).

Aufgrund ihrer leichten Zugänglichkeit sind die tertiären Phosphine III mit gleichen Resten R bevorzugt.

Beispiele für geeignete tertiäre Phosphorverbindungen III sind Triethylphosphin, Tri-n-propylphosphin, Tri-n-butylphosphin, Tricyclohexylphosphin, Tribenzylphosphin, Triphenylphosphin, Trimethylphosphit, Triethylphosphit, Tri-n-propylphosphit, Tri-isopropylphosphit, Tri-n-butylphosphit, Tribenzylphosphit und Triphenylphosphit.

Auf die Art der Reste R kommt es nach den bisherigen Beobachtungen im Prinzip bei den Nitrilen I, tertiären Aminen IIa und tertiären Phosphorverbindungen III nicht an, d.h. alle Komplexe eines bestimmten Metalls weisen im Rahmen der üblichen graduellen Unterschiede die gewünschte Aktivität auf.

Aus wirtschaftlichen Gründen wird man Verbindungen mit einfachen Resten R bevorzugen. Solche einfachen Reste sind vornehmlich die Methyl-, Ethyl-, n-Propyl-, iso-Propyl-, n-Butyl-, und die Phenylgruppe.

Die Reste R können in allen Fällen unsubstituierte Kohlenwasserstoff-Reste sein oder solche, die inerte Substituenten wie Alkoxy-, Aryloxy-, Halogen-, Alkoxycarbonyl- oder Nitrilgruppen tragen. Als Substituenten besonders hervorzuheben sind die Nitrilgruppe und solche mit einem tertiären Stickstoff- oder Phosphoratom. Solche geeigneten Liganden sind etwa N,N,N',N'-Tetramethylethylendiamin, 1,2-Bis-(dimethylphosphino)-ethan oder o-Phthalodinitril. In diesen Fällen handelt es sich somit um Liganden mit mehreren Koordinationsstellen.

Unter diesen mehrfach-funktionellen Liganden sind weiterhin Liganden mit einem polymeren Grundgerüst von Bedeutung. Solche polymeren Liganden sind bekannt oder in bekannter Weise erhältlich (vgl. z.B. US-A 4 145 486).

Bevorzugt sind wegen ihrer besseren Zugänglichkeit solche Polymere, die nur gleiche zur Komplexbildung befähigte Gruppen enthalten, wie Polyacrylnitril, Poly-4-vinylpyridin und Poly(diphenylphosphino)styrol.

In einer bevorzugten Ausführungsform des erfindungsgemäßen Verfahrens lassen sich die Katalysatoren in situ bei der Reaktion erzeugen, wenn man ein Salz eines Platinmetalles und die erfindungsgemäßen Komplexbildner als solche der Reaktionsmischung zusetzt.

Zur Synthese der Katalysatoren wird der Komplexbildner den Platinmetallverbindungen vorzugsweise in bis zu vierfacher stöchiometrischer Menge zugesetzt.

Für diese in-situ-Erzeugung der Katalysatoren geeignete Verbindungen der Platinmetalle sind die Chloride, Nitrate, Acetate und Acetylacetonate bevorzugt. Besonders bevorzugt sind die Chloride.

Beispiele für geeignete Salze sind $PdCl_2$, $Pd(OAc)_2$, $RuCl_3 \cdot x H_2O$ (Ru-Gehalt 40 - 43 Gew.-%), $Ru(acac)_3$, $OsCl_3$

oder $RhCl_3$.

Die Menge der eingesetzten Katalysatoren beträgt im allgemeinen 0,1 bis 10, vorzugsweise 0,5 bis 5 mol-% der Menge der Carboxylgruppen in den zu vinylierenden Carbonsäuren.

Das erfindungsgemäße Verfahren kann unter homogener oder heterogener Katalyse durchgeführt werden. Bevorzugt ist die homogen katalysierte Reaktion, weil der hohe Verteilungsgrad des Katalysators bessere Raum-Zeit-Ausbeuten liefert, als im heterogen katalysierten Fall. Demgemäß eignen sich besonders diejenigen Katalysatoren, die im Reaktionsgemisch unter den Reaktionsbedingungen löslich sind.

Die heterogene Katalyse ist besonders in den Fällen von Bedeutung, in welchen die Liganden an ein polymeres, unlösliches Grundgerüst gebunden sind.

Nach den bisherigen Beobachtungen ist das gute Gelingen des erfindungsgemäßen Verfahrens von der Art der Carbonsäure und von der Anzahl der Carboxylgruppen in der Ausgangsverbindung grundsätzlich nicht abhängig. Es kommen daher ein- und mehrwertige aliphatische, cycloaliphatische, araliphatische und aromatische Carbonsäuren in Betracht, darunter vor allem solche, deren Vinylester für die Herstellung von Kunststoffen besondere Bedeutung haben, wie Essigsäure, Propionsäure und Adipinsäure.

Im gleichen Maße eignen sich als Ausgangsverbindungen für das erfindungsgemäße Verfahren auch substituierte Carbonsäuren, sofern die Substituenten unter den Reaktionsbedingungen inert sind. Solche inerten Substituenten sind Alkoxy-, Aryloxy-, Halogen-, Alkoxycarbonyl- oder Nitrilgruppen. Beispiele für solche substituierten Carbonsäuren sind Bernsteinsäuremonomethylester, Malonsäuremonomethylester, Maleinsäuremonomethylester, Chloressigsäure und 4-Nitrobenzoesäure.

Das erfindungsgemäße Verfahren läßt sich beispielsweise wie folgt in an sich bekannter Weise durchführen:
Man legt die Carbonsäure, gegebenenfalls ein Lösungsmittel und den Katalysator im Reaktor vor, bringt diesen in der Regel zunächst auf die Reaktionstemperatur, gibt gasförmiges Acetylen zu und preßt es in dem Maße, wie es verbraucht wird, nach. Aus Sicherheitsgründen kann man das Acetylen mit einem Inertgas wie Helium, Methan, Propan, Argon oder vorzugsweise Stickstoff verdünnen.

Die Reaktion kann in Gegenwart eines organischen Lösungsmittels durchgeführt werden. Zum Beispiel eignen sich Kohlenwasserstoffe wie n-Pentan, n-Hexan, die Xylole, oder vorzugsweise Toluol, daneben Ether, z.B. 1,4-Dioxan oder Ethylenglykoldimethylether, oder vorzugsweise Tetrahydrofuran, N,N-Dialkylamide, z.B. N-Methyl-2-pyrrolidon oder N,N-Dimethylformamid, sowie Carbonsäureester wie Methylacetat, Ethylacetat oder Butylacetat. In einer bevorzugten Ausführungsform verwendet man das Reaktionsprodukt selbst als Lösungmittel.

Man setzt das Acetylen bezogen auf die umzusetzenden Carboxylgruppen in der Regel im Molverhältnis von 1:1 bis 100:1, vorzugsweise 1,2:1 bis 30:1, besonders bevorzugt von 1,5:1 bis 10:1 ein.

Die Reaktion läßt sich im Prinzip drucklos durchführen, verläuft jedoch auch bis zu Acetylen-Drücken um 25 bar störungsfrei. Zur Erzielung befriedigender Raum-Zeit-Ausbeute wird sie bevorzugt bei Acetylen-Drücken von 15 bis 20 bar durchgeführt.

Die Temperatur der Umsetzung beträgt 50 bis 200, bevorzugt 80 bis 150°C. Besonders bevorzugt ist aufgrund guter Raum-Zeit-Ausbeuten bei gleichzeitig hoher Produktselektivität das Temperaturintervall von 100 bis 120°C.

Die Aufarbeitung des Reaktionsgemisches erfolgt in an sich bekannter Weise, und zwar vorzugsweise durch fraktionierte Destillation oder durch Kristallisation. Der Anteil an Nebenprodukten ist in der Regel sehr gering und besteht hauptsächlich aus Polymerisationsprodukten des Acetylens. Zurückgewonnene Katalysatoren, Lösungsmittel und nicht umgesetzte Ausgangsverbindungen werden zweckmäßigerweise in die Vinylierung zurückgeführt.

Für die bei Reaktionsende im Autoklaven verbleibenden geringen Mengen Acetylen lohnt sich normalerweise die Rückführung in den Syntheseprozeß nicht, und es entfällt der bei anderen Verfahren zu diesem Zweck erforderliche hohe technische Aufwand.

Die nach dem erfindungsgemäßen Verfahren hergestellten Vinylester eignen sich als Monomere oder Comonomere für die Herstellung von Polymerdispersionen, die beispielsweise als wetterbeständige Anstrichmittel dienen.

Beispiele

Eine Mischung aus je 0,2 mol (a Gramm) einer Carbonsäure, b mmol Katalysatormetall (als Salz eines Platinmetalls oder als fertiger Platinmetall-Katalysatorkomplex eingesetzt), c mmol eines Liganden und 120 g Toluol wurden bei 100°C, einem Stickstoffdruck von 5 bar und einem Acetylendruck von 14 bar der Vinylierung unterworfen. Die übliche destillative Aufarbeitung des Reaktionsgemisches lieferte den Vinylester der Carbonsäure in einer Ausbeute von y %. Die Einzelheiten dieser Beispiele sowie deren Ergebnisse sind der Tabelle zu entnehmen.

Tabelle

| Bsp. | Carbonsäure | a [g] | Metall | [mmol] | Ligand | [mmol] | Reaktionsdauer [h] | Vinylester-Ausbeute [%] |
|---|---|---|---|---|---|---|---|---|
| 1 | 2-Ethylhexansäure | 29 | Ru | 2,8 | $NEt_3$ | 6 | 15 | 94 |
| 2 | 2-Ethylhexansäure | 29 | Ru | 6 | CO[1] | | 11 | 80 |
| 3 | 2-Ethylhexansäure | 29 | Ru | 2 | $(n\text{-}Bu)_3P$ | 4 | 7 | 75 |
| 4 | 4-tert.-Butylbenzoesäure | 36 | Ru | 2,5 | $(n\text{-}Bu)_3P$ | 4 | 12 | 89 |
| 5 | Korksäure[2] | 35 | Ru | 2,5 | $(n\text{-}Bu)_3P$ | 4 | 17 | 55 |
| 6 | Bernsteinsäuremonomethylester | 26 | Ru | 2,5 | $(n\text{-}Bu)_3P$ | 4 | 8 | 74 |
| 7 | 2-Ethylhexansäure | 29 | Ru | 2,4 | $(MeO)_3P$ | 4 | 5 | 52 |
| 8 | 2-Ethylhexansäure | 29 | Ru[2] | | [3] | | 13 | 76 |
| 9 | 2-Ethylhexansäure | 29 | Ru | 7,4 | [4] | | 17 | 82 |

1) als $Ru_3(CO)_{12}$

2) Lösungsmittel: 120 g Tetrahydrofuran

3) zurückgewonnener Katalysator aus Bsp. 1

4) $RuCl_3 \cdot x\ H_2O$ aufgezogen auf ein $(CH_3)_2N$-Gruppen tragendes Polystyrol-/Divinylbenzolcopolymerisat; Ru-Anteil: 9,4 Gew.-%

## Patentansprüche

1. Verfahren zur Herstellung von Carbonsäurevinylestern durch Umsetzung einer Carbonsäure mit Acetylen in Gegenwart eines Platinmetallkomplex-Katalysators, dadurch gekennzeichnet, daß man als Platinmetallkomplex-Katalysator einen Komplex aus einem Platinmetall und den Liganden Kohlenmonoxid, ein Nitril (I), eine tertiäre Stickstoffbase (II) oder eine tertiäre Phosphorverbindung mit C- und/oder O-organischen Resten (III) oder Gemischen dieser Liganden verwendet.

2. Verfahren nach Anspruch 1, dadurch gekennzeichnet, daß man als Platinmetall Ruthenium verwendet.

3. Verfahren nach den Ansprüchen 1 und 2, dadurch gekennzeichnet, daß die Liganden in den Katalysatorkomplexen Nitrile der Formel Ia

$$R—CN \qquad (Ia)$$

sind, in der R für eine $C_1$- bis $C_4$-Alkylgruppe, eine Cyclopentyl-, Cyclohexyl-, Phenyl-, Benzyl- oder 2-Phenylethylgruppe steht, wobei diese Reste auch inerte Substituenten tragen können.

4. Verfahren nach Anspruch 1 oder 2, dadurch gekennzeichnet, daß die Liganden in den Katalysatorkomplexen tertiäre Amine der Formel IIa

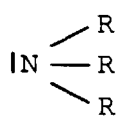

(IIa)

sind, in der die Reste R gleich oder verschieden sein können und die in Anspruch 3 angegebene Bedeutung haben.

5. Verfahren nach Anspruch 1, dadurch gekennzeichnet, daß die Liganden in den Katalysatorkomplexen heteroaromatische Stickstoffbasen (IIb) sind.

6. Verfahren nach Anspruch 5, dadurch gekennzeichnet, daß die heteroaromatischen Stickstoffbasen Pyridin oder 1,10-Phenanthrolin sind.

7. Verfahren nach Anspruch 1 oder 2, dadurch gekennzeichnet, daß die Liganden in den Katalysatorkomplexen tertiäre Phosphorverbindungen mit C- und/oder O-organischen Resten der allgemeinen Formel III

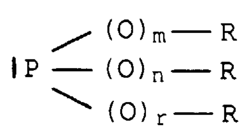

(IIIa)

sind, in der die Reste R gleich oder verschieden sein können und die in Anspruch 3 angegebene Bedeutung haben und in der m, n und r null oder 1 bedeuten.

8. Verfahren nach den Ansprüchen 1 bis 7, dadurch gekennzeichnet, daß man solche Katalysatorkomplexe verwendet, in denen die Liganden I bis III mit einem Polymeren verknüpft sind.

## Claims

1. A process for the preparation of vinyl carboxylates by reacting a carboxylic acid with acetylene in the presence of a platinum metal complex catalyst, wherein the platinum metal complex catalyst is a complex of a platinum metal

and the ligands carbon monoxide, a nitrile (I), a tertiary nitrogen base (II) or a tertiary phosphorus compound containing C- and/or O-organic radicals (III), or a mixture of these ligands.

**2.** A process as claimed in Claim 1, wherein the platinum metal is ruthenium.

**3.** A process as claimed in Claim 1 or 2, wherein the ligands in the catalyst complex are nitriles of the formula Ia

$$R - CN \qquad (Ia)$$

where R is $C_1$- to $C_4$-alkyl, cyclopentyl, cyclohexyl, phenyl, benzyl or 2-phenylethyl, where these radicals may also carry inert substituents.

**4.** A process as claimed in Claim 1 or 2, wherein ligands in the catalyst complex are tertiary amines of the formula IIa

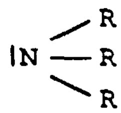

(IIa)

where the radicals R may be identical or different and are as defined in Claim 3.

**5.** A process as claimed in Claim 1, wherein the ligands in the catalyst complex are heteroaromatic nitrogen bases (IIb).

**6.** A process as claimed in Claim 5, wherein the heteroaromatic nitrogen bases are pyridine or 1,10-phenantroline.

**7.** A process as claimed in Claim 1 or 2, wherein the ligands in the catalyst complex are tertiary phosphorus compounds containing C- and/or O-organic radicals, of the formula III

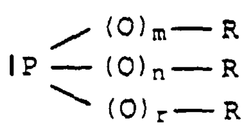

(IIIa)

where the radicals R may be identical or different and are as defined in Claim 3, and m, n and r are zero or 1.

**8.** A process as claimed in any of Claims 1 to 7, wherein the catalyst complexe is one in which the ligands I to III are linked to a polymer.

## Revendications

**1.** Procédé de fabrication d'esters vinyliques d'acides carboxyliques par réaction d'un acide carboxylique avec de l'acétylène en présence d'un catalyseur à base d'un complexe d'un métal du groupe du platine, caractérisé en ce que l'on utilise comme catalyseur à base d'un complexe d'un métal du groupe du platine, un complexe formé d'un métal du groupe du platine et des ligands monoxyde de carbone, un nitrile (I), une base azotée tertiaire (II) ou un composé tertiaire du phosphore à groupements C- et/ou O-organiques (III) ou des mélanges de ces ligands.

**2.** Procédé selon la revendication 1, caractérisé en ce que l'on utilise du ruthénium en tant que métal du groupe du platine.

**3.** Procédé selon l'une des revendications 1 et 2, caractérisé en ce que les ligands des complexes catalyseurs sont des nitriles de formule Ia

$$R—CN \qquad (Ia)$$

dans laquelle R est mis pour un groupement alkyle en $C_1$-$C_4$, cyclopentyle, cyclohexyle, phényle, benzyle ou 2-phényléthyle, ces groupements pouvant porter en outre des substituants inertes.

**4.** Procédé selon l'une des revendications 1 et 2, caractérisé en ce que les ligands des complexes catalyseurs sont des amines tertiaires de formule IIa

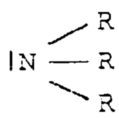

(IIa)

dans laquelle les groupements R peuvent être identiques ou différents et prennent la signification donnée dans la revendication 3.

**5.** Procédé selon la revendication 1, caractérisé en ce que les ligands des complexes catalyseurs sont des bases azotées hétéroaromatiques (IIb).

**6.** Procédé selon la revendication 5, caractérisé en ce que les bases azotées hétéroaromatiques sont la pyridine ou la 1,10-phénanthroline.

**7.** Procédé selon l'une des revendications 1 et 2, caractérisé en ce que les ligands des complexes catalyseurs sont des composés tertiaires du phosphore à groupements C- et/ou O-organiques de formule générale III

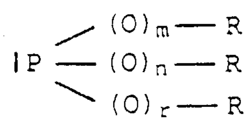

(IIIa)

dans laquelle les groupements R peuvent être identiques ou différents et prennent la signification donnée dans la revendication 3 et dans laquelle m, n et r signifient 0 ou 1.

**8.** Procédé selon l'une des revendications 1 à 7, caractérisé en ce que l'on utilise des complexes catalyseurs dans lesquels les ligands I à III sont liés à un polymère.